# EUROPEAN PATENT APPLICATION

(11) **EP 2 216 004 A1**
(43) Date of publication of application: **11.08.2010**
(21) Application number: 08848412.6
(22) Date of filing: 04.11.2008
(51) Int. Cl.: A61H 1/02, A61B 5/00, A61B 5/0245, A63B 71/06

(54) **APPARATUS FOR EXERCISE THERAPY AND DIAGNOSTIC APPARATUS FOR LOWER LIMB ARTERIAL OBSTRUCTION**

(30) Priority: 07.11.2007 JP 2007289828
(71) Applicant: JMS Co., Ltd., Hiroshima-shi, Hiroshima 730-8652 (JP)
(72) Inventor: SEKII, Hiroyoshi, Wakayama-shi Wakayama 641-0045 (JP); HAYASHI, Shuro, Hatsukaichi-shi Hiroshima 738-0036 (JP); YOSHIMOTO, Mitsuo, Tokyo 125-0063 (JP)
(74) Representative: Collin, Jérôme
(86) International application number: PCT/JP2008/003168
(87) International publication number: WO 2009/060590

(57) **Abstract**

An exercise therapy device 1 includes a pulse wave sensors 16 for detecting a pulse wave of a lower limb of a patient when being fixed to the patient, a walking sensor 15 for detecting a walking distance that the patient has walked, and a target walking distance setting section 30. The target walking distance setting section 30 is configured to set a target walking distance for exercise therapy, when the pulse wave detected by the pulse wave sensors 16 has a waveform having a predetermined shape which is flatter than an arterial pressure waveform, based on the walking distance of the patient by the time when the pulse wave has the waveform having the predetermined shape.

## Description

### TECHNICAL FIELD

The present invention relates to an exercise therapy apparatus for use in exercise therapy for lower extremity arterial occlusive disease and a diagnosis apparatus for lower extremity arterial occlusive disease.

### BACKGROUND ART

With changes in lifestyle habits and the like, the number of patients with chronic lower extremity arterial occlusive diseases such as arteriosclerosis obliterans (ASO), peripheral arterial occlusive disease (PAOD) and the like has increased in recent years. Symptoms of arteriosclerosis obliterans are divided into four groups, i.e., I-IV degrees, according to Fontaine classifications. Symptoms of Fontaine classification I include minor symptoms such as feeling of cold and numbness in the lower limb. As a symptom of Fontaine classification II, after walking a certain distance, the patient feels pain in the lower limb and can no longer walk, but after taking a rest, the pain is relieved and the patient can walk again. This symptom is referred to as intermittent claudication, and a distance from a point at which the patient has started walking to a point where the patient can no longer walk because of pain is referred to as a pain initiation distance (claudication distance). Intermittent claudication is one of symptoms that patients with arteriosclerosis obliterans report most, and has a feature that knotted pain appears around the calf in the lower limb when walking and thus climbing stairs is particularly difficult. In Fontaine classification III, the patient feel strong pain in the lower limb even when the patient is at rest during night and the like. Symptoms of Fontaine classification III include a symptom in which ulcer is formed in the lower limb and necrosis occurs. According to an epidemiological study, the incidence rate of relatively mild cases such as Fontaine classifications I and II per year is approximately 50,000-100,000 per million population, and the incidence rate of relatively severe cases such as Fontaine classifications III and IV is approximately 500-1,000 per million population.

General treatment guidelines for arteriosclerosis obliterans or peripheral arterial occlusive diseases encourage conservative procedures, which are typified by exercise therapy, for mild cases, and revascularization procedures for severe cases. Exercise therapy aims at increasing tolerance to low oxygen conditions by application of an exercise load. To perform exercise therapy, in general, a patient walks. Exercise therapy is directed to improvement in symptoms, i.e., increase of a pain initiation distance to approximately 1.5 times to approximately twice that before treatment within three months from the start of treatment. If further improvement is desired, use of revascularization procedures is considered. Also, in severe cases, as soon as arteriosclerosis obliterans, peripheral arterial occlusive diseases and the like are diagnosed, intravascular treatment using a catheter, or a direct revascularization procedure by a bypass surgery or the like has to be performed as soon as possible. If improvement by such revascularization procedures is not observed, limb amputation is required. Since direct revascularization procedures and limb amputations in severe case are highly invasive, it is desired to find lower extremity arterial occlusive disease in an early stage, and undertake a proper treatment based on determination of the degree of severity before the development of such severe conditions.

When determining the degree of severity of lower extremity arterial occlusive disease, for example, an apparatus described in PATENT DOCUMENT 1 may be used. The apparatus is configured such that a measurement target site of a patient is irradiated with measurement light, transmitted scattering-light from the measurement target site is received by a photodetector, and thereby an oxygen level in body is measured.

### CITATION LIST

### PATENT DOCUMENT

PATENT DOCUMENT 1: Japanese Patent Application No. 2002-136505

### SUMMARY OF THE INVENTION

### TECHNICAL PROBLEM

However, the apparatus of PATENT DOCUMENT 1 is configured such that the oxygen condition in the body of a patient is measured using light, and the degree of transmittance and scattering of light might vary depending on the body constitution of the patient and the like. Thus, the measured oxygen condition does not always correspond to the actual oxygen condition of the patient. Accordingly, there might be cases where the degree of severity of lower extremity arterial occlusive disease cannot be accurately determined.

In order to choose a course of treatment suitable for the degree of severity of a patient, it is important to accurately determine the degree of severity of lower extremity arterial occlusive disease. Particularly, in severe cases where exercise therapy is suitable, an exercise load by walking has to be applied. However, an appropriate exercise load varies depending on the degree of the patient, and thus, if the degree of severity cannot be accurately determined, an appropriate exercise load cannot be applied, so that therapeutic effects cannot be sufficiently obtained.

Some of symptoms of a mild case of lower extremity arterial occlusive disease are similar to symptoms of a nervous system disease. Therefore, it is sometimes difficult to determine lower extremity arterial occlusive disease or a nervous system disease based on only description of pain given by a patient, and thus, patients at risk of lower extremity arterial occlusive diseases might be missed. Furthermore, 20-30% of severe cases of lower extremity arterial occlusive diseases are observed in patients on chronic hemodialysis. In patients under hemodialysis treatment, reduction in circulating blood volume is observed as well as bradycardia, and the lower limb becomes ischemic as the circulating blood volume decreases. Depending on cases, patients with diabetic nephropathy who need hemodialysis have neurological disorders, and, in many cases, such a patient does not feel pain in the lower limb even when the lower limb is ischemic. This prevents early detection of lower extremity arterial occlusive diseases and also is a cause of progression of symptoms in severe lower extremity arterial occlusive diseases.

A first object of the present invention is to support a patient with lower extremity arterial occlusive disease in their exercise therapy, thereby achieving early improvement of symptoms. A second object of the present invention is to allow accurate diagnosis of lower extremity arterial occlusive diseases, thereby realizing early detection and early treatment of the lower extremity arterial occlusive diseases.

### SOLUTION TO THE PROBLEM

To achieve the first and second objects, according to the present invention, pulse waves of a lower limb of a patient are detected for use in exercise therapy or diagnosis.

Specifically, according to a first aspect of the present invention, an exercise therapy apparatus for use in exercise therapy for lower extremity arterial occlusive disease is configured to include a pulse wave detector section for detecting a pulse wave of a lower limb of a patient, a walking distance detector section for detecting a walking distance of the patient, and a target walking distance setting section, to which the pulse wave detector section and the walking distance detector section are connected, for setting, when the pulse wave detected by the pulse wave detector section has a waveform having a predetermined shape which is flatter than an arterial pressure waveform, a target walking distance for exercise therapy, based on the walking distance that the patient has walked by the time when the pulse wave has the waveform.

With this configuration, the pulse wave of the lower limb of the patient is detected by the pulse wave detector section, and the walking distance that the patient has walked is detected by the walking distance detector section.

In general, in a normal case, an observed pulse wave of a limb periphery has a similar shape to the arterial pulse waveform. The present inventors found, as a result of observation of patients with mild cases of lower extremity arterial occlusive disease, that while in a resting state, a pulse wave of a patient has a waveform having a similar shape to the shape of an arterial pressure waveform, a pulse wave of the patient has a waveform having a predetermined shape which is flatter than the arterial pressure waveform, when the patient to whom an exercise load by walking is applied is close to a limit where the patient can no longer walk, and then an ischemic condition occurs. Based on this, during exercise therapy, if the pulse wave detected by the pulse wave detector section has the waveform having the predetermined shape which is flatter than the arterial pressure waveform, it can be understood that the patient is close to the walking limit of the patient. The walking distance that the patient has walked during a period from a time when the patient is in a resting state to a time when the patient is close to a walking limit of the patient decreases, as the severity of lower extremity arterial occlusive disease increases. Thus, the severity of lower extremity arterial occlusive disease can be determined based on the pulse wave and the walking distance. Also, the target walking distance setting section sets the target walking distance for exercise therapy based on the walking distance, and thus, an exercise load according to the severity of the patient can be applied to the patient.

According to a second aspect of the present invention, the exercise therapy apparatus of the first aspect is configured to further include a display section for displaying the target walking distance set by the target walking distance setting section.

With this configuration, the target walking distance set by the target walking distance setting section can be displayed.

According to a third aspect of the present invention, the exercise therapy apparatus of the first or second aspect further includes a notifying section connected to the target walking distance setting section, and is configured to determine whether or not the walking distance of the patient has reached the target walking distance set by the target walking distance setting section and to output, if the walking distance has reached the target walking distance, a notifying signal, and the notifying signal is input to the notifying section.

With this configuration, when the walking distance has reached the target walking distance, the notifying section notifies that the walking distance has reached the target walking distance.

According to a fourth aspect of the present invention, in the exercise therapy apparatus of the third aspect, the notifying section includes at least one of a speaker, a light emitting portion, and a vibration portion.

According to a fifth aspect of the present invention, the exercise therapy apparatus for use in lower extremity arterial occlusive disease according to any one of the first to fourth aspects further includes a remaining distance display section for displaying a remaining distance before the walking distance of the patient reaches the target walking distance set by the target walking distance setting section.

With this configuration, the remaining distance to the target walking distance can be displayed.

According to a sixth aspect of the present invention, the exercise therapy apparatus of any one of the first to fifth aspects is configured so that a memory section for storing the pulse wave detected by the pulse wave detector section and the walking distance detected by the walking distance detector section is provided to an apparatus body.

With this configuration, the pulse waves and walking distance of a patient in the past can be obtained.

According to a seventh aspect of the present invention, a diagnosis apparatus for lower extremity arterial occlusive disease includes a pulse wave detector section for detecting a pulse wave of a lower limb of a patient, and a control section to which the pulse wave detector section is connected, for receiving the pulse wave detected by the pulse wave detector section, and the control section is configured to notify, when the pulse wave detected by the pulse wave detector section has a waveform having a predetermined shape which is flatter than an arterial pressure waveform, that the pulse wave has the waveform having the predetermined shape.

With this configuration, the pulse wave of the lower limb of a patient is detected by the pulse wave detector section, and it is notified, when the pulse wave has a waveform having a predetermined shape which is flatter than an arterial pressure waveform, that the pulse wave has the waveform having the predetermined shape. As described above, when an ischemic condition due to lower extremity arterial occlusive disease occurs, the pulse wave of the lower limb has a waveform having a shape which is flatter than the arterial pressure waveform, and thus, when the patient reports pain in the lower limb, it is possible to determine that the pain is due to lower extremity arterial occlusive disease or neurological disorder. Also, for example, by detecting the pulse wave of the lower limb during hemodialysis, it is possible to determine whether or not an ischemic condition due to lower extremity arterial occlusive disease has occurred in the lower limb even if the patient does not report pain. Thus, lower extremity arterial occlusive disease can be found in an early stage.

### ADVANTAGES OF THE INVENTION

According to the first aspect of the present invention, the target walking distance for exercise therapy is set, when the pulse wave detected by the pulse wave detector has a waveform having a predetermined shape which is flatter than an arterial pressure waveform, based on the walking distance that the patient has walked by the time when the pulse wave has the waveform. Thus, an exercise load according to the severity of the patient can be applied to the patient, so that therapeutic effects can be sufficiently achieved.

According to the second aspect of the present invention, the display section for displaying the target walking distance is provided. Thus, the patient or a healthcare professional can easily recognize the target walking distance.

According to the third aspect of the present invention, when the walking distance of the patient has reached the target walking distance, it can be notified by the notrifier section that the walking distance of the patient has reached the target walking distance. Thus, it is possible to notify the patient or the healthcare professional that the patient has walked the target walking distance.

According to the fourth aspect of the present invention, the notifying section includes at least one of a speaker, a light emitting portion, and a vibration portion. Thus, it is possible to reliably notify the patient or the healthcare professional that the patient has walked the target walking distance.

According to the fifth aspect of the present invention, the remaining distance display section for displaying the remaining distance is provided. Thus, the patient or the healthcare professional can easily recognize the remaining distance to the target walking distance.

According to the sixth aspect of the present invention, the pulse wave and the walking distance can be stored in the memory section. Thus, by referring to stored data after walking, the degree of severity of symptoms can be easily recognized.

According to the seventh aspect of the present invention, it is notified, when the pulse wave detected by the pulse wave detector section has a waveform having a predetermined shape which is flatter than an arterial pressure waveform, that the pulse wave has the waveform having the predetermined shape. Thus, lower extremity arterial occlusive disease can be found, and also accurately diagnosed in an early stage, so that early treatment can be undertaken

### BRIEF DESCRIPTION OF THE DRAWINGS

[FIG. 1] FIG. 1 is a view illustrating an exercise therapy apparatus for lower extremity arterial occlusive disease according to a first embodiment when being in use.
[FIG. 2] FIG. 2 is a schematic view of the exercise therapy apparatus.
[FIG. 3] FIG. 3 is a block diagram of the exercise therapy apparatus.
[FIG. 4] FIG. 4 is a schematic view of a part of a fixing belt.
[FIG. 5] FIG. 5 is a flowchart showing the operation of the exercise therapy apparatus.
[FIG. 6] FIG. 6(a) is a graph showing a normal pulse wave. FIG. 6(b) is a graph showing a pulse wave of a patient with lower extremity arterial occlusive disease when the lower limb of the patient is ischemic.
[FIG. 7] FIG. 7 is a schematic view of an exercise therapy apparatus according to a first variation of the first embodiment, corresponding to FIG. 2.
[FIG. 8] FIG. 8 is a schematic view of an exercise therapy apparatus according to a second variation of the first embodiment, corresponding to FIG. 2.
[FIG. 9] FIG. 9 is a view of an exercise therapy apparatus according to a third variation of the first embodiment, corresponding to FIG. 1.
[FIG. 10] FIG. 10 is a view of an exercise therapy apparatus according to a fourth variation of the first embodiment, corresponding to FIG. 1.
[FIG. 11] FIG. 11 is a block diagram of a diagnosis apparatus for lower extremity arterial occlusive disease according to a second embodiment.

### DESCRIPTION OF REFERENCE CHARACTERS

- 1: Exercise Therapy Apparatus for Lower extremity arterial occlusive disease
- 2: Apparatus Body
- 3: Fixing Band (Fixing Section)
- 10: CPU
- 15: Walking Sensor
- 16: Pulse Wave Sensor
- 19: Lamp (Notifying Section)
- 20: Speaker (Notifying Section)
- 21: Vibrator (Notifying Section)
- 23: Distance Display Section (Remaining Distance Display Section)
- 24: Storage Section
- 50: Diagnosis Apparatus for Lower Extremity Arterial Occlusive Disease

### DESCRIPTION OF EMBODIMENTS

Embodiments of the present invention will be described hereinafter in detail with reference to the accompanying drawings. The following embodiments have been set forth merely for purposes of preferred examples in nature, and are not intended to limit the scope, applications, and use of the invention.

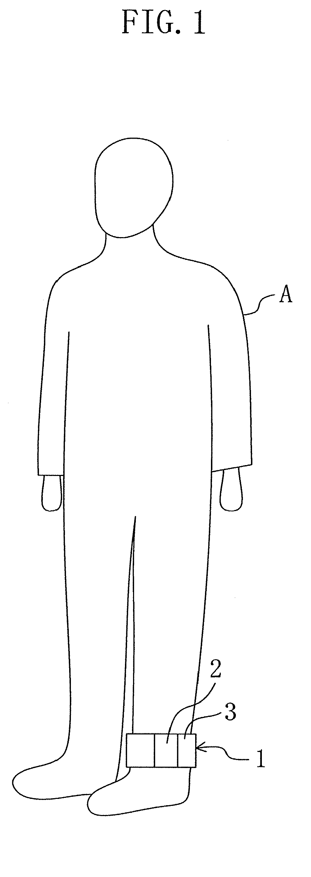

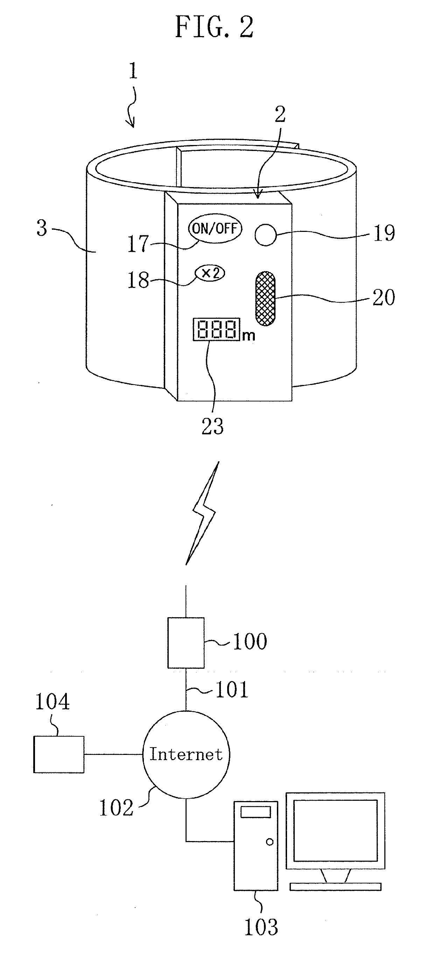

FIG. 1 is a view illustrating an exercise therapy apparatus 1 for lower extremity arterial occlusive disease according to a first embodiment of the present invention when being in use. The exercise therapy apparatus 1 is used when a patient A with lower extremity arterial occlusive disease performs an exercise therapy and, as shown in FIG. 2, includes an apparatus body 2 and a fixing band (fixing section) 3 for fixing the apparatus body 2 to a lower limb of the patient A, for example, around an ankle of the patient A. The fixing band 3 includes a hook-and-loop fastener or the like, and is configured not to be unhooked while being placed around the lower limb. The apparatus body 2 may be fixed using, for example, a belt or the like, instead of the fixing band 3.

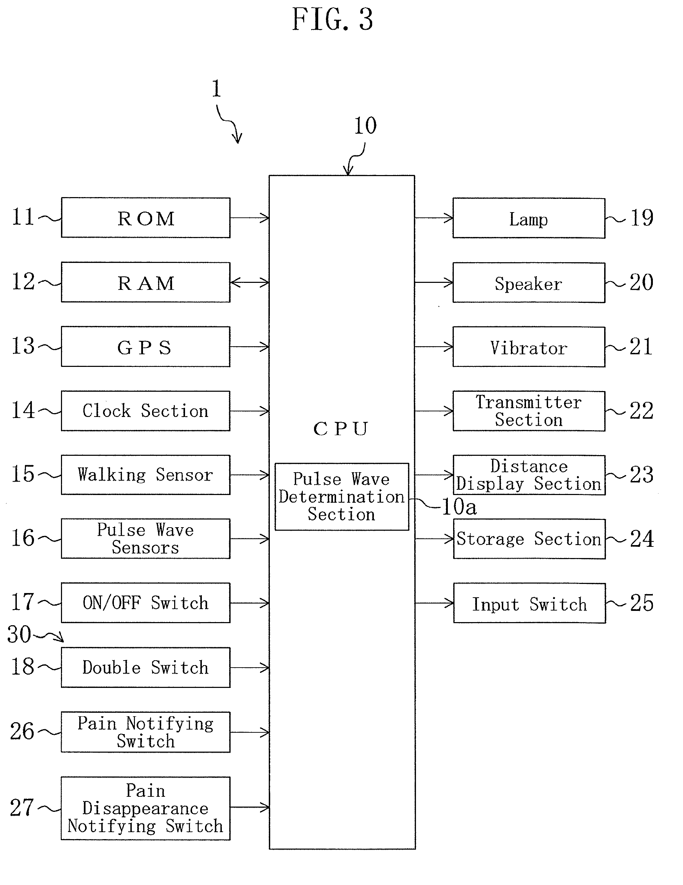

As shown in FIG. 3, the apparatus body 2 includes a CPU 10. A ROM 11, a RAM 12, a GPS 13, a clock section 14, a walking sensor 15, a pulse wave sensor (pulse wave detector section) 16, an ON/OFF switch 17, a double switch 18, a lamp (illumination section) 19, a speaker 20, a vibrator (vibration portion) 21, a transmitter 22, a distance display section 23, a storage section 24, an input switch 25, a pain notifying switch 26, and a pain disappearance notifying switch 27 are connected to the CPU 10 via signal lines. The CPU 10, the ROM 11 and the RAM 12 serve as a control section of this invention. Although not illustrated in the drawings, the apparatus body 2 includes a rechargeable battery as a power source. Note that the apparatus body 2 may be configured to include a dry cell battery or a solar cell battery, instead of the rechargeable battery.

In the ROM 11, a program for operating the exercise therapy apparatus 1 is stored. In the RAM 12, data retrieved while the exercise therapy apparatus 1 is in operation is continuously stored during the operation of the apparatus 1. The GPS 13 is a global positioning system having a known configuration which receives a radio wave transmitted from a predetermined artificial satellite to calculate a current position. A calculation result of current position by the GPS 13 is continuously output as positional data to the CPU 10. The clock section 14 is configured to continuously output current date (i.e., year, month, and day) and time to the CPU 10.

The walking sensor 15 includes a biaxial acceleration sensor to detect whether or not the patient A is walking. The walking sensor 15 has a similar configuration to that of a walking counter mounted on a known digital pedometer or the like. The walking sensor 15 is configured to output a pulse wave for each step. The walking sensor 15 is not limited to the above-described acceleration sensor, but a sensor which detects change in plantar pressure, a sensor which detects change in electrical potential using expansion and contraction of metal wires attached to both legs, trousers or the like of the patient A, or like sensor may be used as the walking sensor 15.

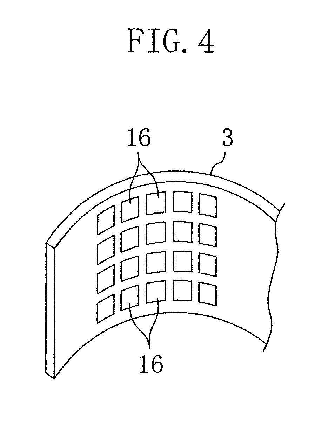

The pulse wave sensors 16 are comprised of known pressure detection sensors (for example, a piezoelectric sensors) and, as shown in FIG. 4, are attached to the fixing band 3. The pulse wave sensors 16 are provided so that each of the pulse wave sensors 16 touches the skin of a part around the angle of the patient A and thus pulse waves from an artery running under the skin can be noninvasively detected. The pulse wave sensors 16 may be provided to touch any part of the lower limb where a pulse wave can be detected. For example, for an obese patient with a thick fat layer, the pulse wave sensors 16 may be arranged to touch the dorsum or bottom of the foot, or the like. The pulse wave sensors 16 preferably touch a part of the body at which a healthcare professional can check the pulse of the patient by feeling the part, but a part which the pulse wave sensors 16 touch is not limited to the above-described part.

The pulse wave sensors 16 are arranged in a plurality of arrays in the lateral direction of the fixing band 3 and also in a plurality of arrays in the longitudinal direction of the fixing band 3. Thus, a large detection range in which a pulse wave can be detected can be ensured, and therefore, even when the position of the fixing band 3 being fixed is slightly shifted, a pulse wave can be detected. The CPU 10 is configured to use signals of at least one of the pulse wave sensors 16, whose a detection value regularly varies. The arrangement of the pulse wave sensors 16 is not limited to the above-described arrangement. For example, the pulse wave sensors 16 may be arranged in lines only in the lateral direction or only in the longitudinal direction, or in a lattice pattern. The number of the pulse wave sensors 16 may be one.

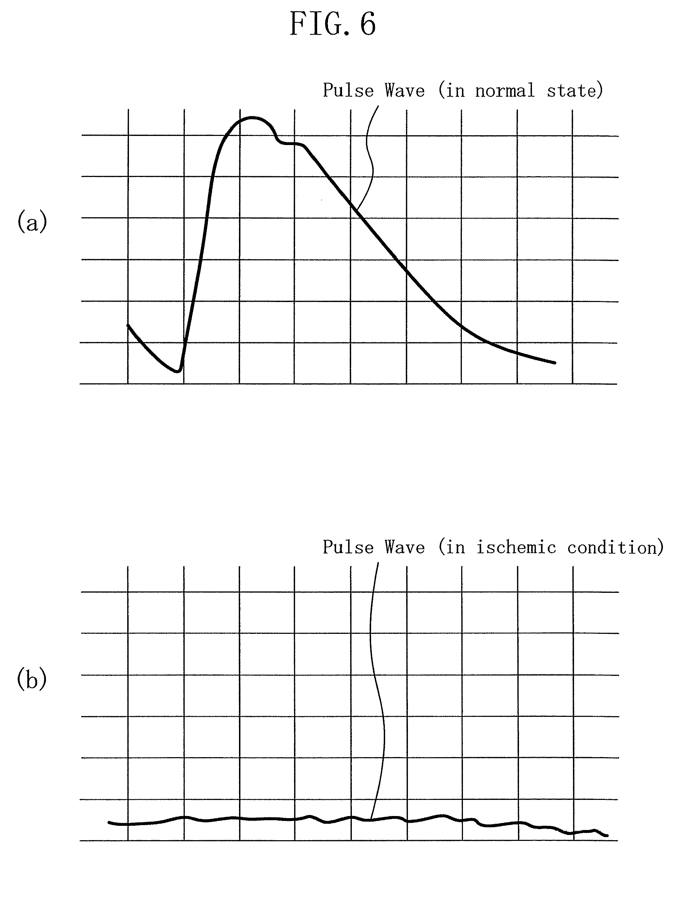

A pulse wave detected by the pulse wave sensors 16 will be described with reference to FIG. 6(a). A pulse wave is comprised of a percussion wave generated by contraction of the heart and a tidal wave generated by reflection of the percussion wave at a peripheral artery, an arterial branch and the like when the percussion wave runs through the entire body. In a normal case, a pulse wave of the lower limb having a similar shape to an arterial pressure waveform is detected.

The ON/OFF switch 17 is a switch for turning ON and OFF the power of the apparatus body 2. The double switch 18 is a switch for setting a target walking distance. The pain notifying switch 26 is a switch operated by the patient A when pain appears in the lower limb while the patient A is walking. The pain disappearance notifying switch 27 is a switch operated by the patient A when pain that has once appeared disappears.

The lamp 19 lights up when the patient A can no longer walk because pain appears (the patient A has walked to a pain initiation distance), and when the walking distance of the patient A has reached a target walking distance. Similarly, the speaker 20 generates a certain sound when the patient A has walked to the pain initiation distance, and when the walking distance of the patient A has reached the target walking distance. Similarly, the vibrator 21 vibrates when the patient A has walked to the pain initiation distance, and when the walking distance of the patient A has reached the target walking distance. The vibrator 21 is comprised of a known electrical vibrator. The lamp 19, the speaker 20, and the vibrator 21 serve as a notifying section of the present invention. The lamp 19 may be configured to maintain a light-up state or flicker.

The storage section 24 includes a storage medium such as a known memory card or the like, and is removable from the apparatus body 2. Data such as date, time and place at which exercise therapy is performed using the exercise therapy apparatus 1, walking path, pulse wave during walking, pain initiation distance, time taken for a patient to reach the pain initiation distance since the start of walking, target walking distance, time taken for a patient to reach the target walking distance since the start of walking, date and time and walking distance at which the pain notifying switch 26 is pressed, date and time and walking distance at which the pain disappearance notifying switch 27 is pressed, break time if a patient rests during walking, a recovering time from a time when pain appears to a time when the pain disappear, and the like is stored in the storage section 24. The place at which exercise therapy is performed and the walking path can be obtained by the GPS 13.

The transmitter section 22 transmits the data stored in the storage section 24 to a terminal equipment 104 (shown in FIG. 2) such as a personal computer operated, for example, by a healthcare professional, and includes a known transmitter and an antenna. To transmit data, as shown in FIG. 2, the transmitter section 22 accesses the Internet 102 using a known receiving terminal 100 such as a cellular phone or a PHS, and a communication network 101. The data is stored in a patient data server 103 of a medical institute via the Internet 102. The data transmitted to the patient data server 103 is processed to be in form required by the healthcare professional or an administrator, and then, is transmitted to the terminal equipment 104 of the healthcare professional or the administrator. The form required by the healthcare professional or the administrator herein is a graph form to represent numerical values or the like.

The distance display section 23 displays the pain initiation distance and the remaining distance to the target walking distance, and includes a digital indicator. The input switch 25 is for inputting a length of stride of the patient A to the apparatus body 2.

The CPU 10 operates according to a program stored in the ROM 11. The CPU 10 is configured so that, as an initial setting, the length of stride of the patient A is input thereto by the input switch 25. The CPU 10 obtains the number of steps based on pulse waves input from the walking sensor 15, and calculates the walking distance by multiplying the number of steps by the length of stride. The walking sensor 15 and the CPU 10 serves as a walking distance detector section of the present invention.

Calculation of the walking distance starts when the power is turned ON by the ON/OFF switch 17 and then input of pulse waves from the walking sensor 15 is started. The calculated walking distance is output as data from the CPU 10 to the distance display section 23 and is displayed by the distance display section 23. The walking distance can be obtained also by the GPS 13 and, if the length of stride is not input at a time of initial setting, a moving distance obtained by the GPS 13 is used as the walking distance to control the exercise therapy apparatus 1. If the GPS 13 is used, the moving distance of the patient A can be used as the walking distance, regardless of the length of stride, and thus, an accurate walking distance can be obtained even when the length of stride is changed during walking. Moreover, by using the GPS 13, the place and walking path where the patient A has walked can be accurately identified.

As shown in FIG. 3, a pulse wave determination section 10a for determining whether or not a pulse wave of the lower limb input from the pulse wave sensors 16 have a waveform having a predetermined shape which is flatter than an arterial pressure waveform. The pulse wave determination section 10a is configured to store a pulse wave (shown in FIG. 6(a)) in a normal state (in a resting state) detected by the pulse wave sensors 16 and determines, when a pulse wave (shown in FIG. 6(b)) whose peaks are much lower than a peak of the pulse wave in a normal state amount is detected by the pulse wave sensors 16, that a current pulse wave of a patient has the waveform having the predetermined shape. This determination method utilizes the characteristic of the pulse wave of a patient A with lower extremity arterial occlusive disease, which the present inventors found. That is, in general, in normal case, a pulse wave of a limb periphery having a similar shape to the arterial pulse waveform is observed. However, a pulse wave of a patient with a mild case of lower extremity arterial occlusive disease has a similar shape to an arterial pressure waveform when the patient is in a resting state, but when the patient with an exercise load by walking applied is close to a limit where the patient can no longer walk and an ischemic condition occurs, the pulse wave has a waveform which is flatter than the arterial pressure waveform. This means that when it is determined by the pulse wave determination section 10a that the pulse wave has the waveform having the predetermined shape, the patient can no longer walk because of pain. Note that a "waveform having a predetermined shape" used herein means a shape whose peaks are lower than a peak of the pulse wave in a resting state by a certain amount and which allows determination of the occurrence of an ischemic condition of the lower limb based on a pulse wave, and is not limited to a single waveform. The waveform having the predetermined shape may be a plurality of different shapes. The peaks of the waveform having the predetermined shape is preferably 1/5 or less of the peak of the pulse wave in a resting state, and more preferably 1/10 or less of the peak. The waveform having the predetermined shape may be changed for each patient A, or the same waveform may be commonly used for a plurality of patients.

When it is determined by the pulse wave determination section 10a that a pulse wave has the waveform having the predetermined shape, the CPU 10 stops calculation of the walking distance. A distance which a patient has walked during a time from a time when walking is started to a time when it is determined that the pulse wave has the waveform having the predetermined shape is stored as the pain initiation distance in the RAM 12, and also, a time from a time when walking is started to a time when it is determined that the pulse wave has the waveform having the predetermined shape is stored as a time (a pain initiation distance time) taken for the patient to walk the pain initiation distance in the RAM 12. The CPU 10 is configured to output a notifying signal when it is determined by the pulse wave determination section 10a that the pulse wave has the waveform having the predetermined shape. The lamp 19 which has received the notifying signal flickers, the speaker 20 generates a predetermined sound, and the vibrator 21 is vibrated. Thus, it is notified that the pulse wave has the waveform having the predetermined shape.

When the pulse wave determination section 10a determines that the pulse wave has the waveform having the predetermined shape and then the double switch 18 is pressed, the pain initiation distance is doubled, and the doubled distance is obtained as a target walking distance. The target walking distance is output to the distance display section 23 from the CPU 10 to be temporarily displayed by the distance display section 23.

The CPU 10 is configured to start calculation of the walking distance in the above-described manner when, after the double switch 18 being pressed, the start of a pulse wave input from the walking sensor 15 is detected. Also, the CPU 10 is configured to obtain a remaining distance to the target walking distance by deducting the walking distance of the patient from the target walking distance, and then output the remaining distance to the distance display section 23. The distance display section 23 is also used as a remaining distance display section.

Furthermore, the CPU 10 is configured to output a notifying signal when an entire walking distance has reached the target walking distance. The lamp 19, the speaker 20, and the vibrator 21 are operated in response to the notifying signal. Thus, it is notified that the entire walking distance of the patient A has reached the target walking distance.

That is, the CPU 10 and the double switch 18 serve as a target walking distance setting section 30 (see FIG. 3) of the present invention, so that, when the pulse wave detected by the pulse wave sensors 16 has the waveform having the predetermined shape which is flatter than the arterial pressure waveform, the target walking distance for exercise therapy can be set based on the walking distance of the patient who has walked by the time when the pulse wave has the waveform. The target walking distance may be automatically set by the CPU 10. Specifically, after the pulse wave determination section 10a determines that the pulse wave has the waveform having the predetermined shape, the CPU 10 doubles a distance which the patient has walked by then to set the doubled distance as the target walking distance. Thus, the patient A do not have to operate the double switch 18.

When the CPU 10 detects that the pain notifying switch 26 is pressed before the patient reaches the target walking distance, the CPU 10 causes the RAM 12 to store the time and walking distance at which the pain notifying switch 26 is pressed. Furthermore, when, after the pain notifying switch 26 being pressed, the CPU 10 detects that the pain disappearance notifying switch 27 is pressed, the CPU 10 causes the RAM 12 to store the time and walking distance at which the pain disappearance notifying switch 27 is pressed.

If the patient A stops walking, output of a pulse wave from the walking sensor 15 stops, and thereby the CPU 10 can detect that the patient A has stopped walking. The CPU 10 causes the RAM 12 to store the time at which the patient A has stopped walking and the distance which the patient A has walked by then. Then, when detecting that the patient A restarts walking, the CPU 10 causes the RAM 12 to store the time at which the patient A has restarted walking. To detect that the patient A has stopped walking and restarted walking, the GPS 13 may be used, or a motion sensor (acceleration sensor) may be additionally provided and used for the detection.

The CPU 10 is provided with an automatic adjusting function for adapting the exercise therapy apparatus 1 to the patient A. For each patient A, the shape of peaks of the pulse wave differs, and the condition of skin also differs. Therefore, if a sensitivity for pulse wave detection is the same at any time, there might be cases where even if the sensitivity is suitable for a patient A, the sensitivity is so high for another patient A that noise becomes noticeable, or conversely, the sensitivity is so low for another patient A that a pulse wave cannot be detected. The automatic adjusting function is configured to detect, before the exercise therapy apparatus 1 is attached, a pulse wave of an artery (for example, a radial artery) in an upper limb (for example, the wrist) and set the detection sensitivity to be relatively low for a patient A whose pulse wave is easily identified, and relatively high for a patient A whose pulse wave is difficult to be identified. Even in a patient A with lower extremity arterial occlusive disease, a pulse wave can be more easily identified in the upper limb, as compared to the lower limb, and therefore, the upper limb is more suitable for detection sensitivity adjustment.

The CPU 10 is configured to cause the RAM 12 to store, as a break time, a time from a time wheh output of pulse waves from the walking sensor 15 is stopped to a time when output of pulse waves from the walking sensor 15 is restarted. The pulse wave sensors 16 is configured to continuously perform pulse wave detection even when pulse waves are not output from the walking sensor 15. Furthermore, the CPU 10 causes the RAM 12 to store, as a recovering time, a time taken for a patient to recover to be in a state in which the waveform of the pulse wave is back to the waveform in a resting state from a time when the pulse wave has the waveform having the predetermined shape.

The data stored in the RAM 12 is stored in the storage section 24.

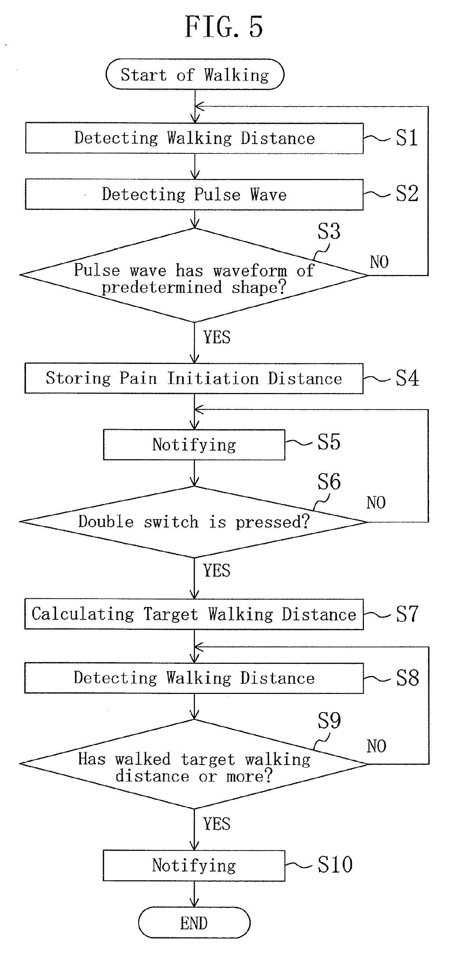

Next, usage of the exercise therapy apparatus 1 having the above-described configuration will be described with reference to a flowchart shown in FIG. 5. When the exercise therapy apparatus 1 is used, first, the apparatus body 2 is fixed to the lower limb of a patient A using the fixing band 3. In this case, the pulse wave sensors 16 is held against the skin and, in this state, the fixing band 3 is tied. Then, the ON/OFF switch 17 is pressed to turn the power ON. Also, the length of stride of a patient A is measured, and then the measured length of stride is input to the apparatus body 2 using the input switch 25. Furthermore, a pulse wave in a resting state is detected, and is stored in the RAM 12 by performing a predetermined operation of the input switch 25.

After input of the length of stride, when the patient A starts walking, an operation proceeds to Step S1 in the flowchart to detect the walking distance of the patient A. Specifically, in Step S1, in the CPU 10, a distance obtained by multiplying the number of pulse waves input from the walking sensor 15 by the length of stride is set as the walking distance. The walking distance is displayed by the storage section 24. Also, the walking start time, place, and walking path are stored in the RAM 12. In the subsequent to Step S1, i.e., Step S2, the pulse wave detected by the pulse wave sensors 16 is input to the CPU.

Thereafter, the operation proceeds to Step S3 to determine whether or not the pulse wave detected in Step S2 has a predetermined waveform which is flatter than a pulse wave in a resting state stored in the RAM 12.

In Step S3, if peaks of the pulse wave are higher than a predetermined value and the pulse wave does not have the predetermined waveform, a determination of NO is made, and then the operation returns to Step S1 and proceeds again to Step S2 and then to Step S3. When the determination in Step S3 is NO, the lower limb of the patient A has not yet become ischemic, and thus, the patient A do not feel strong pain during walking and can continue to walk.

In Step S3, if the determination is YES, which indicates that the pulse wave has the waveform having the predetermined shape, the operation proceeds to Step S4. When the determination in Step S3 is YES, the lower limb of the patient A has become ischemic, and thus, the patient A feels strong pain and has difficulty in walking. The pain initiation time is a time from a time when walking is started to a time when a determination of YES is made in Step S3, and is stored in the RAM 12.

In Step S4 to which the operation proceeds in response to the determination of YES made in Step S3, a distance that the patient has walked during a time from a time when walking is started to a time when a determination of YES is made in Step S3 is stored as the pain initiation distance in the RAM 12. If the severity of the patient A's condition is high, the patient A cannot walk a long distance at a time, as compared to a case in which the degree of severity is low, and thus, the pain initiation distance decreases as the degree of severity of the patient increases. In this case, when the patient A presses the pain notifying switch 26, the information that the pain notifying switch 26 is pressed is temporarily stored in the RAM 12 with the time and the walking distance at which the pain notifying switch 26 is pressed.

Thereafter, the operation proceeds to Step S5 to cause the lamp 19 to flicker, the speaker 20 to generate a sound, and the vibrator 21 to vibrate, thereby notifying that the pulse wave has the waveform having the predetermined shape. In subsequent Step S6, whether or not the double switch 18 is pressed is determined. If it is determined that the double switch 18 is not pressed in Step S6, a determination of NO is made, the operation returns to Step S5, and notification is continuously performed. If the double switch 18 is pressed and the determination is YES, the operation proceeds to Step S7, and the target walking distance for exercise therapy is calculated. The target walking distance is obtained by doubling the pain initiation distance. Since the pain initiation distance is determined according to the degree of severity of the patient A, the target walking distance is determined according to the degree of severity of the patient A. When the double switch 18 is pressed, the notification is stopped. In Step S4, notification may be performed by using only one of the lamp 19, the speaker 20, and the vibrator 21, or any two of them.

The patient A rests for a while, and restarts walking after pain in the lower limb is decreased. A time before the patient A restarts walking is a break time and is stored in the RAM 12. When the pain is decreased and the patient A presses the pain disappearance notifying switch 27, the information that the pain disappearance notifying switch 27 is pressed is temporarily stored in the RAM 12 with the time and the walking distance at which the pain disappearance notifying switch 27 is pressed. Since pulse wave detection by the fixing band 3 is performed even while the patient A is not walking, it is possible to recognize that, when the shape of the pulse wave becomes close to the shape of the pulse wave in a resting state, the pain of the patient A has almost disappeared. Thus, the recovering time can be obtained and is stored in the RAM 12. If the patient A does not restart walking even after the shape of the pulse wave has become close to the shape of the pulse wave in a resting state, it is presumed that the patient A is not walking because the patient is waiting for the traffic light to change, or for some other reason.

In the subsequent step to Step S7, i.e., Step S8, the walking distance of the patient A is obtained in the same manner as in Step S1. In subsequent Step S9, whether or not the patient A has walked the target walking distance or more is determined. In Step S9, if the determination is NO, which indicates that the patient A has not yet walked the target walking distance, the operation returns to Step S8.

If the patient A has walked the target walking distance or more and the determination is YES in Step S9, the operation proceeds to Step S10. In Step S10, the lamp 19 is caused to flicker, the speaker 20 is caused to generate a sound, and the vibrator 21 is caused to vibrate, thereby notifying the patient A and people around the patient A that the patient A has walked the target walking distance or more. That is, the patient A has to walk until the entire walking distance reaches a distance which is twice as long as the pain initiation distance even with breaks when pain appears. Thus, the patient A has increased tolerance to low oxygen conditions because of effects of exercise load. This exercise therapy may be performed, for example, three times a week for three or more consecutive months. If symptoms of the patient A are improved, the pain initiation distance is increased, and also, the target walking distance is increased. These distances are displayed by the distance display section 23, and the patient A can recognize the degree of improvement in symptoms by seeing data in numeric form, and thus can realize therapeutic effects.

When the patient A performs exercise therapy using the exercise therapy apparatus 1, it is recoded in the storage section 24 that the exercise therapy is performed using the exercise therapy apparatus 1. Data recorded in the storage section 24 is transmitted to the patient data server 103 via the Internet 102 by the transmitter section 22. The data transmitted to the patient data server 103 is transmitted to the terminal equipment 104 of a healthcare professional. Thus, the healthcare professional can specifically recognize the degree of improvement in symptoms of the patient A by checking data represented in graph form or in numeric form. The storage section 24 may be removed from the apparatus body 2 and connected to the patient data server 103 so that data in the storage section 24 is directly transmitted to the patient data server 103.

As has been described, the exercise therapy apparatus 1 of this embodiment is configured so that, when the pulse wave detected by the pulse wave sensors 16 has the waveform having the predetermined shape, the target walking distance for exercise therapy is set based on the walking distance of the patient A by the time when the pulse wave has the waveform having the predetermined shape. Thus, an exercise load according to the severity of the patient A can be applied to the patient, so that therapeutic effects can be sufficiently achieved.

The distance display section 23 for displaying the target walking distance is provided to the apparatus body 2, and thus, the patient A and a healthcare professional can easily recognize the target walking distance.

When the patient A has walked the target walking distance, it can be notified by the lamp 19, the speaker 20, and the vibrator 21 that the patient A has walked the target walking distance, and thus, the patient A and the healthcare professional can be notified that the patient A has walked the target walking distance.

The distance display section 23 of the apparatus body 2 is configured to display the remaining distance to the target walking distance, and thus, the patient A and the healthcare professional can easily recognize the remaining distance.

Pulse wave data and walking distance data can be stored in the storage section 24 of the apparatus body 2 and transmitted to the patient data server 103, and thus, the healthcare professional can easily recognize the degree of improvement in symptoms by checking the data.

In this embodiment, the pain initiation distance is doubled and the doubled distance is set as the target walking distance. However, the target walking distance is not limited thereto. The target walking distance may be obtained by multiplying the pain initiation distance by a smaller multiplying factor than 2, i.e., for example, 1.5, or may be obtained by multiplying the pain initiation distance by a larger multiplying factor than 2, i.e., for example, 2.5. The multiplying factor may be set to a plurality of values in a stepwise fashion, may be set not in a stepwise fashion, or may be changed for each patient A.

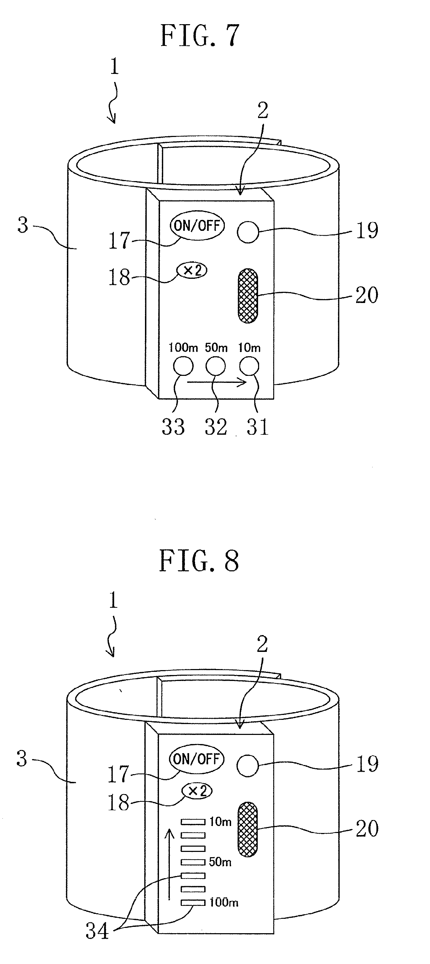

Moreover, as in a first variation shown in FIG. 7, first through third lamps 31, 32 and 33 for displaying an estimate of the remaining distance before reaching the target walking distance may be provided to the exercise therapy apparatus 1. The first lamp 31 is a green lamp and lights up when the remaining distance is, for example, 10 m or less. The second lamp 32 lights up when the remaining distance is 10 m or more and 50 m or less. The third lamp 33 lights up when the remaining distance is 50 m or more and 100 m or less. The lamps 31 through 33 are connected to the CPU 10. The CPU 10 is configured to cause only the first lamp 31 to light up when the remaining distance is 10 m or less, only the second lamp 32 to light up when the remaining distance is 50 m or less and 10 m or more, and only the third lamp 33 to light up when the remaining distance is 100 m or less and 50 m or more. The remaining distances at which the first through third lamps 31 through 33 light up are not limited to the above-described distances, but may be arbitrarily set. The color of each of the lamps 31 through 33 may be arbitrarily set.

As in a second variation shown in FIG. 8, a plurality of bar-type lamps 34 may be provided to light up according to the remaining distance as in the first variation. In the first and second variations, the distance display section 23 may be omitted. Also, the remaining distance may be notified by a sound generated by the speaker 20.

In the first embodiment, three portions of the notifying section, i.e., the lamp 19, the speaker 20, and the vibrator 21 are provided to the device body 2. However, only one or two of the lamp 19, the speaker 20 and the vibrator 21 may be provided thereto.

Exercise therapy using the exercise therapy apparatus 1 may be performed under the supervision of a healthcare professional, or may be performed by a patient A at home. Depending on symptoms of the patient A, some other therapy such as medication therapy or the like may be performed along with exercise therapy.

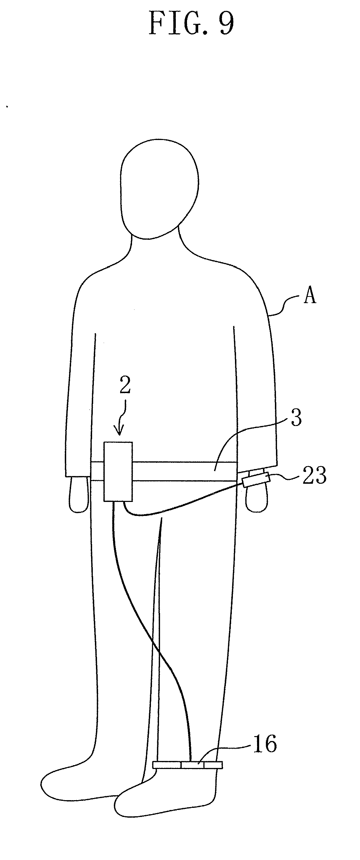

In the first embodiment, the pulse wave sensors 16 is integrated with the walking sensor 15 or the GPS 13, and is configuration to be fixed to the lower limb. However, as in a third variation shown in FIG. 9, the pulse wave sensors 16, the CPU 10 and the GPS 13, and the distance display section 23 may be formed as separate portions so that only the pulse wave sensors 16 can be fixed at the lower limb, the CPU 10 and the GPS 13 can be fixed to the waist, and the distance display section 23 can be fixed to the arm. In this case, the CPU 10 and the pulse wave sensors 16 may be connected together via a wired connection, or via a wireless connection. As an example of the wireless connection, a short range wireless connection with a communication distance of about 10 m is preferable, and, for example, Bluetooth or the like can be used.

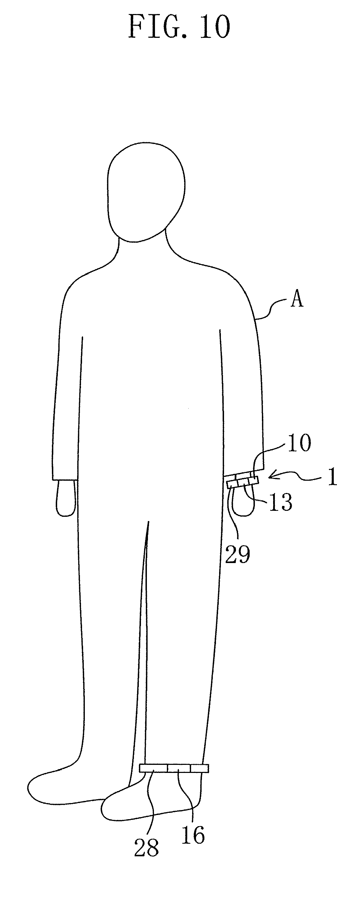

As in a fourth variation shown in FIG. 10, only the pulse wave sensors 16 may be fixed to the lower limb by a fixing band 28, and other components such as the CPU 10, the GPS 13 and the like may be fixed to the wrist by a fixing band 29. Similarly to the third variation, in this case, the CPU 10 and the pulse wave sensors 16 may be connected via a wired connection, or via a wireless connection.

### (Second Embodiment)

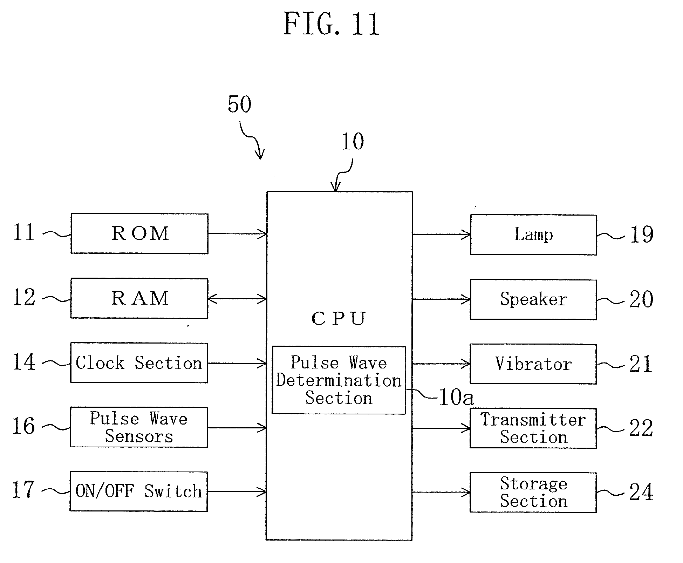

FIG. 11 is a block diagram of a diagnosis apparatus 50 for lower extremity arterial occlusive disease according to a second embodiment of the present invention. Except that the GPS 13, the walking sensor 15, the double switch 18, the pain notifying switch 26 and the pain disappearance notifying switch 27 of the exercise therapy device 1 of the first embodiment are not provided, main components of the diagnosis apparatus 50 are the same as those of the exercise therapy apparatus 1 of the first embodiment. Therefore, like or similar elements are designated by the same reference character, the description thereof will be omitted, and only different elements will be described in detail.

The CPU 10 is configured to output a notifying signal to the lamp 19, the speaker 20, and the vibrator 21 when a pulse wave detected by the pulse wave sensors 16 has a waveform having a predetermined shape which is flatter than an arterial pressure waveform of a patient A. The lamp 19 which has received the notifying signal flickers, the speaker 20 generates a predetermined sound, and the vibrator 21 vibrates. That is, the CPU 10 is configured to notify, when the pulse wave detected by the pulse wave sensors 16 has the waveform having the predetermined shape which is flatter than the arterial pressure waveform, people around the apparatus that the pulse wave detected by the pulse wave sensors 16 has the waveform having the predetermined shape. The CPU 10 serves as a control section of the present invention.

Next, usage of the diagnosis apparatus 50 is used will be described. The diagnosis apparatus 50 is attached around the ankle of a patient A in the same manner as in attaching the exercise therapy apparatus 1 of the first embodiment. In the diagnosis apparatus 50, an arterial pressure waveform of the patient A is stored beforehand. The arterial pressure waveform can be obtained from an artery at the upper limb.

The ON/OFF switch 17 is pressed to turn the power ON. Then, a pulse wave of a lower limb is detected by the pulse wave sensors 16, and then input to the CPU 10. If the pulse wave does not have the waveform having the predetermined shape, the CPU 10 does not output a notifying signal, and thus, the lamp 19, the speaker 20, the vibrator 21 are not operated. If the pulse wave has the waveform having the predetermined shape, the CPU 10 outputs a notifying signal, and thus, the lamp 19, the speaker 20, and the vibrator 21 are operated, thereby notifying the patient A and people around the apparatus that the lower limb has become ischemic.

Accordingly, by using the diagnosis apparatus 50, it is possible to determine, when the patient A reports pain in the lower limb, that the pain is due to lower extremity arterial occlusive disease or neurological disorder. Also, for example, by detecting the pulse wave of the lower limb during hemodialysis, it is possible to determine whether or not the lower limb has become ischemic even if the patient A does not reports pain. Thus, lower extremity arterial occlusive disease can be found in an early stage.

Therefore, by using the diagnosis apparatus 50 of the second embodiment, it is possible to notify, when the pulse wave of the lower limb has a waveform having a predetermined shape which is flatter than an arterial pressure waveform, that the pulse wave has the waveform having the predetermined shape. Thus, lower extremity arterial occlusive disease can be accurately diagnosed in an early stage, so that early treatment can be undertaken.

In the second embodiment, three portions of the notifying section, i.e., the 19, the speaker 20, and the vibrator 21 are provided to the apparatus body 2. However, only one or two of the lamp 19, the speaker 20 and the vibrator 21 may be provided thereto.

Also, the exercise therapy apparatus 1 of the first embodiment may be used in diagnosis of lower extremity arterial occlusive disease, which has been described in the second embodiment. In this case, it is preferable to configured the exercise therapy apparatus 1 so that a program for the diagnosis apparatus 50 of the second embodiment is incorporated in the ROM 11 of the exercise therapy apparatus 1 beforehand, and the program for exercise therapy and the program for diagnosis can be changed over. However, the configuration of the exercise therapy apparatus 1 is not limited thereto.

### INDUSTRIAL APPLICABILITY

As has been described, an exercise therapy apparatus and a diagnosis apparatus for lower extremity arterial occlusive disease according to the present invention can be used in treatment and diagnosis of arteriosclerosis obliterans and peripheral arterial occlusive diseases.

## Claims

1. An exercise therapy apparatus for use in exercise therapy for lower extremity arterial occlusive disease, comprising:
a pulse wave detector section for detecting a pulse wave of a lower limb of a patient;
a walking distance detector section for detecting a walking distance of the patient; and
a target walking distance setting section, to which the pulse wave detector section and the walking distance detector section are connected, for setting, when the pulse wave detected by the pulse wave detector section has a waveform having a predetermined shape which is flatter than an arterial pressure waveform, a target walking distance for exercise therapy, based on the walking distance that the patient has walked by the time when the pulse wave has the waveform.

2. The apparatus of claim 1, further comprising:
a display section for displaying the target walking distance set by the target walking distance setting section.

3. The apparatus of claim 1 or 2, further comprising:
a notifying section connected to the target walking distance setting section, and
wherein
the apparatus is configured to determine whether or not the walking distance of the patient has reached the target walking distance set by the target walking distance setting section and to output, if the walking distance has reached the target walking distance, a notifying signal, and
the notifying signal is input to the notifying section.

4. The apparatus of claim 3, wherein
the notifying section includes at least one of a speaker, a light emitting portion, and a vibration portion.

5. The apparatus of any one of claims 1-4, further comprising:
a remaining distance display section for displaying a remaining distance before the walking distance of the patient reaches the target walking distance set by the target walking distance setting section.

6. The apparatus of any one of claims 1-5, further comprising:
a memory section for storing the pulse wave detected by the pulse wave detector section and the walking distance detected by the walking distance detector section.

7. A diagnosis apparatus for lower extremity arterial occlusive disease, comprising:
a pulse wave detector section for detecting a pulse wave of a lower limb of a patient; and
a control section to which the pulse wave detector section is connected, for receiving the pulse wave detected by the pulse wave detector section,
wherein
the control section is configured to notify, when the pulse wave detected by the pulse wave detector section has a waveform having a predetermined shape which is flatter than an arterial pressure waveform, that the pulse wave has the waveform having the predetermined shape.
